# EUROPÄISCHE PATENTSCHRIFT

(11) **EP 2 260 829 B1**
(45) Veröffentlichungstag und Bekanntmachung des Hinweises auf die Patenterteilung: **11.05.2016**
(21) Anmeldenummer: 10405065.3
(22) Anmeldetag: 26.03.2010
(51) Int. Cl.: A61Q 19/08, A61K 8/97, A61K 36/05

(54) **Verwendung eines Extraktes aus Schneealgen in kosmetischen oder dermatologischen Formulierungen**
Use of an extract from snow algae in cosmetic or dermatological formulations
Utilisation d'un extrait composé d'algues de neige dans des formulations cosmétiques ou dermatologiques

(30) Priorität: 12.06.2009 CH 9172009
(43) Veröffentlichungstag der Anmeldung: 15.12.2010
(73) Patentinhaber: Mibelle AG, 5022 Buchs (CH)
(72) Erfinder: Schürch, Stutz Dr., Cornelia, 5603 Staufen (CH); Schmid, Dr., Daniel, 5200 Brugg (CH); Zülli, Dr., Fred, 5024 Küttigen (CH)
(74) Vertreter: Rottmann, Maximilian

(56) Entgegenhaltungen:
- WO-A1-00/24369
- WO-A1-85/01655
- DE-A1- 19 756 677
- FR-A1- 2 728 467
- US-A1- 2004 225 167
- US-A1- 2009 142 370
- DATABASE GNPD [Online] Mintel; 1. Juli 2006 (2006-07-01), Tibetan Saffron: "After Sun Ice Spring Water Masks - Crystal Collagen Mask", XP002648895, Database accession no. 559245
- THOMAS LEYA ET AL: "Response of arctic snow and permafrost algae to high light and nitrogen stress by changes in pigment composition and applied aspects for biotechnology", FEMS MICROBIOLOGY ECOLOGY, Bd. 67, Nr. 3, 1. März 2009 (2009-03-01), Seiten 432-443, XP55002197, ISSN: 0168-6496, DOI: 10.1111/j.1574-6941.2008.00641.x
- DUVAL B.SHETTY K.THOMAS WH.: "Phenolic compounds and antioxidant properties in the snow alga Chamydomonas nivalis after exposure to light", J. APPL. PHYCOL., Bd. 11, 20. Dezember 2002 (2002-12-20), Seiten 559-566, XP55002198,

## Beschreibung

Die vorliegende Erfindung betrifft die Verwendung eines Extraktes aus Schneealgen, insbesondere aus *Chlamydocapsa* sp. (Schneealgen) in kosmetischen und/oder pharmazeutischen Erzeugnissen. Insbesondere betrifft die Erfindung die Verwendung eines Extraktes aus Schneealgen, insbesondere aus *Chlamydocapsa* sp. (Schneealgen) in kosmetischen und/oder pharmazeutischen Produkten, die zum Schutz vor extrinsischer Hautalterung bedingt durch negative Umwelteinflüsse, wie zum Beispiel UV-Strahlung oder Luftverschmutzung, aber auch zum Schutz vor intrinsischer Hautalterung durch Beeinflussung von alterungsspezifischen Genexpressions-Levels, eingesetzt werden. Die Erfindung betrifft des weiteren ein Verfahren zur Herstellung von Extrakten aus Schneealgen, die für topische Applikationen geeignet sind.

Die Haut ist eine Barriere, die unseren Körper vor der Austrocknung bewahrt und vor chemischen sowie mechanischen Einflüssen der Umwelt schützt. Als besonders schädigend für die Haut gilt die Ultraviolett-Strahlung des Sonnenlichts. Wie jedes Gewebe, ist auch die Haut der Alterung unterworfen. Bei der Haut unterscheidet man zwischen intrinsischer und extrinsischer Alterung. Die intrinsische Alterung ist ein chronologischer, genetisch bedingter Prozeß. Äußere Faktoren bestimmen den extrinsischen Alterungsprozeß. Als besonders schädlich gelten Rauchen und starke Sonnenexposition. Diese extrinsischen Faktoren führen zur so genannten vorzeitigen Hautalterung.
Die Alterung der Haut ist nicht nur ein kosmetisches Problem. Gealterte Haut bedeutet eine reduzierte Barriere-Funktion und damit verminderter Schutz gegen Umwelteinflüsse. Ein verhängnisvoller Kreislauf setzt ein und die Haut altert noch schneller.
Gealterte Haut ist gekennzeichnet durch eine verminderte Zellregeneration in der Oberhaut und einen starken Rückgang in der Produktion von Kollagen-Struktureiweißen in der Lederhaut. Die Haut verliert an Elastizität und ihr Wassergehalt nimmt ab.
Es existieren bereits verschiedene kosmetische wie dermatologische Ansätze zur Behandlung der Hautalterung. Der hauptsächlich eingesetzte Wirkstoff in dermatologischen Produkten ist Retinsäure. In kosmetischen Produkten werden neben UV-Filtern vor allem Antioxidantien, wie Vitamin C oder Vitamin E, eingesetzt. Auch Extrakte von Pflanzen, Algen oder Pilzen, die reich an natürlichen Antioxidantien sind, werden als Wirkstoffe in kosmetischen Mitteln bereits eingesetzt. US2009/0142370 bzw. WO00/24369 offenbaren solche kosmetischen Mittel. Besonders vielversprechende und interessante Substanzen enthalten Pflanzen oder Mikroorganismen, die extremen äußeren Bedingungen ausgesetzt sind. Derartige Organismen, die in der Wüste, an den Erdpolen oder in heißen Quellen leben, gelten als extremophil. Sie mußten für ihr Überleben in diesen extremen Habitaten spezielle Anpassungstechniken entwickeln.
Eine besondere Art von Extremophilen sind die Schneealgen, die biologisch zur Kryoflora gerechnet werden.
Als Algen werden im Wasser lebende, eukaryotische, pflanzenartige Lebewesen bezeichnet, die Photosynthese betreiben, jedoch nicht zu den eigentlichen Pflanzen gehören. Schneealgen gehören zu den Grünalgen und werden systematisch in die Klasse der Chlorophyceae, Division Chlorophyta eingeteilt.

Es handelt sich um mikroskopische Süßwasseralgen, die auf den ewigen Schnee- und Gletscherflächen der polaren und alpinen Regionen unserer Erde vorkommen (Arktis, Antarktis, Alaska, Grönland, West- und Ostküste Nordamerikas, Himalaya, Japan, Neu Guinea, Europa, Schweiz, China, Patagonien, Chile und South Orkney Inseln). In ihrem natürlichen Habitat herrschen extrem tiefe Temperaturen, hohe UV-Strahlung und Nährstoffmangel vor, woran sie sich optimal angepaßt haben. Sie leben in einem einzigartigen Mikrohabitat, dem freien Wasser zwischen den Schneekristallen. Auf den großen Schneeflächen in den polaren und alpinen Regionen führen sie zu Grün- und den besonders auffälligen Rotfärbungen (Blutschnee, z. B. *Chlamydomonas nivalis),* die auf die Sporen der Algen zurückzuführen sind. Die Rotfärbung der Sporen dient dem UV-Schutz und wird durch die Einlagerung von Carotinoiden (Astaxanthin) erzielt.

Es sind zur Zeit etwa 350 Arten von Schneealgen bekannt. Die am weitesten verbreitete Art ist *Chlamydomonas nivalis.* Die phylogenetische Einteilung der Schneealgen ist schwierig und deren Systematik ist in ständigem Wandel. Viele der 350 Arten stammen aus Wildsammlungen und die taxonomische Charakterisierung der Algen auf genetischer Ebene ist noch immer lückenhaft. Die größte Schneealgenstammsammlung befindet sich im Fraunhofer Institut für Biomedizinische Technik (IBMT) in Berlin (CCCryo, Culture Collection of Cryophilic Algae).

Zunehmendes Interesse finden die Schneealgen auch aus ökologischer Sicht. Die Schneealgen können aus der Atmosphäre CO₂ absorbieren und so den Treibhauseffekt beeinflussen. Ebenso dienen sie als Bioindikatoren zum Beispiel für die UV-Einstrahlung, was direkt mit der Abnahme der Ozonschicht zusammenhängt, oder die säuretolerante Arten als Indikator für sauren Regen.

Schneealgen werden im allgemeinen in polaren Gebieten gesammelt, in Laboren gereinigt, kultiviert und auf DNA Ebene taxonomisch bestimmt, siehe hierzu Leya, T. (2004): Feldstudien und genetische Untersuchungen zur Kyrophilie der Schneealgen Nordwestspitzbergens, Shaker Verlag, Aachen. Heutzutage ist es möglich die Schneealgen in entsprechenden Reaktoren dauerhaft zu kultivieren. Die Schwierigkeit der Kultivierung besteht in der Temperaturführung, der Luftzufuhr und der Lichtzufuhr. Besonders der Temperatur muß besondere Aufmerksamkeit geschenkt werden, da die Schneealgen als Kryoflora ein Wachstumsoptimum bei ca. 12°C aufweisen. Der normale Lebenszyklus einer Schneealge beginnt mit der Keimung im Frühjahr, wenn der Schnee langsam schmilzt und genügend Wasser und Nährstoffe zur Verfügung stehen. Die grünen Algen betreiben mit Hilfe von Chloroplasten Photosynthese, wachsen und vermehren sich bis eine erneute Nährstofflimitierung die Sporulation auslöst. Die Sporulation beginnt damit, dass die Algen Carotinoide produzieren und einlagern, die die Sporen vor UV-Strahlung schützen. Die roten Algensporen bedingen damit die typische Rotfärbung des Schnees.
Zur Zeit liegt das Hauptinteresse bei der Erforschung der Schneealgen in der Isolation verschiedener sekundär Metabolite aus den Schneealgen, wie zum Beispiel Carotinoide (Xanthin, Astaxanthin usw.), cryoaktive Enzyme/Proteine (Cryoprotektoren z. B.) oder Antioxidantien, wie sie beispielsweise von Bohne, F. & Linden H. (2002): Regulation of carotenoid biosynthesis genes in response to light in Chlamydomonas reinhardtii. Biochim. Biophys. Acta 1579:26-34, Duval B., Shetty K. & Thomas WH. (2000): Phenolic compounds and antioxidant properties in the snow alga Chamydomonas nivalis after exposure to light, J. Appl. Phycol. 11:559-566, Fäbregas J., Otero A., Mased A. & Dominguez A. (2001): Two-stage cultures for the production of astxanthin form Haemotococcus pluvalis, J. Biotechnol. 89:65-71, bzw. Leya T. et al. (2009): Response of arctic snow and permafrost algae to high light and nitrogen stress by changes in pigment composition and applied aspects for biotechnology, FEMS Microbiol. Ecol. 67:432-443 beschrieben werden.
So beschreibt beispielsweise die US 2004/225167 A1 ein Verfahren zur Herstellung von Carotinoiden, Zeaxanthin aus grünen Chlorphyta-Algen, wobei insbesondere ein Verfahren beschrieben wird, mit dem ein in einem Detergenz-unlöslichen Mittel Carotinoid hergestellt wird. Die WO 2008/141757 A1 bzw. die EP 1 995 325 A1 beschreiben ebenfalls ein Verfahren zur Herstellung von Astaxanthin aus Haematococcus pluvialis, mit denen das Carotionoid in großen Mengen erzeugt werden kann. Eine industrielle Anwendung der Inhaltsstoffe der Schneealgen ist bisher nicht bekannt.

Vor allem die kontinuierliche Kultivierung der Schneealgen, die Aufarbeitung der Biomasse und die Isolation von Zellbestandteilen stellen eine große Herausforderung dar. Die Kultivierung von Schneealgen und die Isolierung einzelner sekundärer Metabolite werden unter anderen in der KR 20000072316 A oder der JP 0908772 beschrieben. Die US 2008/254056 A1 beschreibt die Kultivierung von Grünalgen der Spezies *Haematococcus pluvialis* und die Isolation von Astaxanthin. Die Anwendung der Extrakte beschränkt sich ebenfalls auf die isolierten Substanzen wie Astaxanthin oder Lutein.

Daneben werden Extrakte oder Isolate aus Organismen, die extremem UV-Stress oder Wasser-Streß (Trockenheit; Gefrieren) ausgesetzt sind, ebenfalls bereits als Wirkstoffe für Anti-Aging Kosmetika eingesetzt. Beispiele solcher Wirkstoffe sind das Ectoin und Extrakte mit Mycosporin-ähnlichen Aminosäuren (MAA). Das Ectoin ist ein niedermolekularer Schutzstoff aus der Gruppe der Osmolyte oder kompatiblen Solute. Das Ectoin wird von halophilen Bakterien, die in Salzwüsten leben, produziert. Es schützt die Zellen vor Hitze und Trockenheit. Die DE 199 11 775 A1 beschreibt den Einsatz von Ectoin in kosmetischen Formulierungen zum Schutz der DNA in den Hautzellen vor UV-Strahlung und anderen DNA-schädlichen Faktoren. Die DE 100 55 558A1 beschreibt kosmetische Mittel zur Hautpflege, die einen Extrakt der Grünalge Prasiola crispa antarctica enthalten. Die Alge ist in der Antarktis beheimatet und produziert UV-absorbierende Substanzen (Mycosporin-ähnliche Aminosäuren).

Aufgabe der vorliegenden Erfindung ist es, ein kosmetisches und/oder pharmazeutisches Produkt zum Schutz, zur Behandlung und zur Pflege der Haut bereitzustellen. Insbesondere soll ein kosmetisches und/oder pharmazeutisches Produkt zur Verhinderung oder Verzögerung der Hautalterung bereitgestellt werden, mit dem sowohl die intrinsische als auch die extrinsische Hautalterung behandelt werden kann.

Diese Aufgabe wird erfindungsgemäß durch einen Extrakt aus Schneealgen, insbesondere aus *Chlamydocapsa* sp. gelöst, der in einem kosmetischen und/oder pharmazeutischen Präparat enthalten ist, wobei dieser Extrakt nach einem reproduzierbaren Verfahren mittels einer 2-Phasen-Cryo-Kulitivierung hergestellt wird.

Mit dem erfindungsgemäß verwendeten Extrakt, (hergestellt beispielsweise aus *Chlamydocapsa* sp. (Schneealgen)) dessen hydrophile und lipophile Zellbestandteile als auch die Cytosole liposomal verkapselt und pulverförmig vorliegen, konnte ein positiver Einfluß auf mehrere Parameter der Hautalterung gezeigt werden. Im Besonderen schützt der Extrakt vor extrinsischen Faktoren, die den Hautalterungsprozeß vor allem durch oxidative Reaktionen beschleunigen (PhotoAging zum Beispiel), und gleichzeitig schützt der Extrakt ebenfalls vor intrinsischer Hautalterung, die durch eine veränderte Genexpression in älterer Haut bedingt ist. Dieser kombinierte Schutz ist ein neuartiger Ansatz zur ganzheitlichen Anti-Aging Behandlung im kosmetischen Bereich.

Das Verfahren zur Herstellung eines erfindungsgemäß verwendeten Extraktes aus Schneealgen ist gekennzeichnet durch die Schritte:
Kultivierung von Schneealgen beispielsweise des Stammes *Chlamydocapsa* sp. (CCCryo 101-99, Stammsammlung IBMT) in einem Röhren-Reaktorsystem mit einer 2-Phasen-Kultiverung, wobei die Ernte in der 2. Phase (Rotphase) erfolgt,
Herstellung eines Extraktes, wobei die kultivierten Zellen mittels Hochdruckhomogenisierung aufgeschlossen und anschließend die Inhaltstoffe extrahiert werden und mit leeren Liposomen stabilisiert werden, um fein dispergierte Liposomen zu erhalten, die die fettlöslichen und wasserlöslichen Fraktionen der Zellen enthalten,
Aufbringen und Trocknen des Grundextraktes auf ein Trägermaterial mittels Wirbelschichttrocknung.

Nachfolgend wird die Wirkungsweise der kosmetischen und/oder pharmazeutischen Zusammensetzung beschrieben, die den Schneealgenextrakt nach dem oben beschriebenen Verfahren enthält.

### 1. Schutz vor intrinsischer Hautalterung

Der Extrakt wurde an Alterungsmodellen basierend auf menschlichen Fibroblasten Hautzellen untersucht. Im ersten Modell wurden alte Fibroblastenzellen (natürlich gealtert; nach vielen Teilungen) mit jungen Zellen verglichen.
Im anderen Modell wurden Fibroblasten Zellen durch H₂O₂-Behandlung frühzeitig gealtert. Zur Validierung der Modelle wurde die Expression von Markergenen der Hautalterung analysiert. Eine Anti-Aging Wirkung ist erwiesen, wenn ein Stoff die alterungsbedingte Über- oder Unterexprimierung eines Gens neutralisieren kann. Die Untersuchung an natürlich gealterten Fibroblasten Zellen, die nachfolgend in Beispiel 8 untersucht werden, zeigte, dass ein Extrakt von Schneealgen die Expression der Gene für die Kollagene des Typs I und III stimuliert. Die Kollagene des Typs I und III sind die hauptsächlichen Strukturproteine in der Haut. Gealterte Haut ist gekennzeichnet durch einen reduzierten Gehalt an diesen Kollagenproteinen. Im gleichen Test wurde auch eine starke Stimulierung der Expression des Klotho-Gens gefunden. Das Klotho-Protein agiert als Korezeptor von Rezeptoren der Fibroblasten-Wachstumsfaktoren und reguliert als sekretierte Form andere Wachstumsfaktoren- wie Insulin und IGF-1. Defekte im Klotho-Gen führen zu einem Phänotyp der frühzeitigen Alterung. Durch Überexprimierung von Klotho konnte die Lebensspanne von Mäusen verlängert werden. Entsprechend war in der hier beschriebenen Untersuchung die Expression von Klotho in unbehandelten alten Zellen verglichen mit jungen Zellen reduziert. Durch Behandlung der alten Zellen mit dem Extrakt von Schneealgen konnte die Reduzierung nicht nur neutralisiert sondern sogar überkompensiert werden. Im Test mit den frühzeitig gealterten Fibroblasten, der nachfolgend in Beispiel 9 beschrieben wird, zeigte ein Extrakt von Schneealgen eine Hemmung der Expression der Gene für die Matrix-Metalloproteinase 1 und 3. Beide Proteinasen sind in gealterter Haut in höherer Konzentration vorhanden. Sie sind verantwortlich für den Abbau der Strukturproteine Kollagen und Elastin und so von zentraler Rolle bei der Hautalterung. Die Behandlung mit dem Extrakt von Schneealgen konnte die altersbedingte Überexprimierung der Matrix-Metalloproteinase neutralisieren.

### 2. Schutz vor extrinsischer Hautalterung:

Die hier beschriebene *in vivo* Wirksamkeit konnte in einem Probandentest bestätigt werden, der ausführlich in Beispiel 10 beschrieben wird. Eine Creme mit 3 Gew.-% Schneealgenwirkstoff wurde im Gesicht in einem Halbseitentest geprüft. Das Testprodukt wurde von 18 Frauen und 2 Männern während 3 Wochen zweimal täglich aufgetragen. Das Plazeboprodukt wurde jeweils auf der anderen Gesichtshälfte aufgetragen. Alle Probanden verbrachten die 2. Woche in den Skiferien. Während dieser Zeit war ihr Gesicht extremen Umwelteinflüssen ausgesetzt in Form von Kälte, Trockenheit und hoher UV-Bestrahlung. Die Resultate zeigten, dass die mit dem Schneealgenwirkstoff behandelte Gesichtshälfte besser gegen transepidermalen Wasserverlust (TEWL) geschützt war verglichen mit der Plazeboseite. Die Ausprägung der Gesichtsfalten vor und nach der Behandlung wurde mittels einer VisioFace® Apparatur untersucht. Das ist eine Einrichtung, die es ermöglicht, unter kontrollierten Bedingungen hochauflösende Digitalbilder des ganzen Gesichts zu machen. Die zu verschiedenen Zeiten gemachten Bilder können mit einer Software verglichen werden. Das Resultat zeigte eine signifikante Reduktion der Falten nach der Behandlung mit der Schneealgencreme. Auf der Placeboseite konnte keine signifikante Veränderung festgestellt werden.

Die Herstellung geeigneter Extrakte aus Schneealgen umfaßt die folgenden Hauptschritte:
a) Massenkultivierung der Zellen in einem Röhren-Reaktorsystem und
b) Herstellung eines Gesamtextraktes mittels Hochdruckhomogenisation unter Verwendung von leeren Liposomen und
c) Gleichzeitiges Aufbringen und Trocknen des Extraktes auf geeignetes Trägermaterial mittels Wirbelschichttrocknung.

Die Massenkultivierung der Zellen (a) wird nach bestehenden Protokollen bei einem pH Wert von 5,5 und einer Temperatur von 4°C bis 15°C in gängigen Reaktoren durchgeführt.

Zweckmäßigerweise folgt man beim Schritt b) dem folgenden Verfahrensablauf:
- Aufschluß der Zellen durch Hochdruckhomogenisation;
- Extraktion und Stabilisierung von Inhaltsstoffen mit Liposomen;
wobei beide Teile des Verfahrensablaufs in einem einzigen Verfahrensschritt gleichzeitig durchgeführt werden.

### Herstellung der Biomasse

Für die Herstellung des in dieser Erfindung erwähnten Schneealgenextraktes wird zunächst Biomasse produziert durch Kultivierung von Schneealgen der Spezies Chlamydocapsa, im Spezifischen des Kultivars 101-99 (CCCryo 101-99 Stammsammlung IBMT). Die Produktion der Biomasse in einem geeigneten Röhrenreaktorsystem bei spezifischen Kultivierungsbedingungen erfolgt nach gängigen Methoden. Die Kultivierung wird in zwei Phasen durchgeführt, dies wird ausführlich in Beispiel 1 beschrieben. Nach der 1. Wachstumsphase wird eine 2. Phase angehängt, die durch limitierte Nährstoffzufuhr induziert wird. Dadurch gehen die Schneealgen von der vegetativen, grünen Phase über in die stationäre, rote Phase welche durch die Produktion von Sekundärmetaboliten wie zum Beispiel verschiedenen Carotinoiden gekennzeichnet ist.

Nach dem Ende der Kultivierung wird die Biomasse vom Kultivierungsmedium abgetrennt, tiefgefroren und bei -20°C gelagert bis zur weiteren Verarbeitung.

### Aufarbeitung der Biomasse

Bei dem beschriebenen Verfahren für die Herstellung des kosmetischen Wirkstoffes aus der Schneealgenbiomasse können verschiedenartige Zusätze beigemengt werden, wie zum Beispiel Konservierungsmittel, Antioxidantien, pH-Stabilisatoren, Duftstoffe oder weitere Substanzen, die im Präparat oder im kosmetischen Produkt von Nutzen sind.

Als Konservierungsmittel können alle für Kosmetika zugelassenes Konservierungsmittel natürlichen oder synthetischen Ursprungs, wie beispielsweise Phenoxyethanol, Benzoesäure, Propionsäure, Alkohol oder Silberchlorid, verwendet werden.

Um den Extrakt zusätzlich vor Oxidation zu schützen, können Antioxidantien, wie beispielsweise Ascorbinsäure oder Tocopherol, beigemengt werden.

Nachdem alle Komponenten zusammengefügt wurden, muß die Mischung verrührt werden, um das Konservierungsmittel, sowie andere Zutaten zu lösen. Das kann beispielsweise mit einem Blattrührer, einem Homogenisierungsstab oder durch Pumpen durch statische Mischelemente geschehen.

Die nachfolgende Hochdruckhomogenisation, die in Beispiel 2 ausführlich beschrieben ist, verfolgt zwei Ziele:
- Zerstörung der Zellmembranen, damit die extrahierbaren Stoffe freigesetzt werden, und
- Generierung von fein dispergierten Liposomen, welche die fettlösliche und wasserlösliche Fraktion der Zellen enthalten.

Geeignete Hochdruckhomogenisatoren sind kommerziell auf dem Markt erhältlich. Das Prinzip der Reaktionskammer muß aus verschiedenen Möglichkeiten ausgewählt und vorgängig getestet werden. Ebenso muß die Anzahl der Durchläufe durch die Reaktionskammer bis alle Zellwände aufgeschlossen oder eine gewünschte Homogenität des Extraktes erreicht ist, getestet werden.

Der so erhaltene Extrakt kann danach direkt in ein kosmetisches Produkt, wie beispielsweise Cremen, Seifen, Lotionen, Gele oder Haarseren, eingearbeitet werden oder mittels Wirbelschichttrocknung in eine physikalisch sehr stabile Pulver-Form mit langer Haltbarkeit gebracht werden. Durch die Wirbelschichttrocknung steht die gesamte Oberfläche der einzelnen Partikel in der Flugphase der Trocknung zur Verfügung. Dabei kommt es durch die kontinuierliche und vollständige Durchmischung zu einer homogenen Produkttemperatur und zu einem gleichmäßigen Abtrocknen des Ausgangsprodukts. Eine derartige Trocknung ist sehr effektiv, schnell und schonend. Das Verfahren muß jeweils für die entsprechenden Ausgangsprodukte und die gewünschten Eigenschaften des Endproduktes, Granulat, Pellet, Pulver oder andere, in Bezug auf Temperatur und Luftstrom angepaßt und optimiert werden, dies wird ausführlich in Beispiel 3 beschrieben.

Ebenfalls ist es möglich topische Zubereitungen mit dem erfindungsgemäßen Schneealgenextrakt bereitzustellen. In diesen Zusammensetzungen sind die Wirkstoff in feiner Verteilung vorhanden und werden dadurch bevorzugt in einer durch die Haut resorbierenden Form auf die Haut aufgebracht. Hierzu eignen sich insbesondere Gele, Emulsionen oder Mikroemulsionen.

Falls der Extrakt als Halbfabrikat dienen soll, ist eine zusätzliche Verdickung denkbar. Als Verdickungsmittel können dabei alle für Kosmetika erlaubten natürlichen und synthetischen Verdicker oder Verdickungsmittel, wie beispielsweise Xanthangummi, Hyaluronsäure, Carrageen, Dextrin, modifizierte Stärke oder Agar, eingesetzt werden.

Ein bevorzugter erfindungsgemäß verwendeter Scheealgen-Extrakt, der in einer kosmetische und/oder pharmazeutische Zusammensetzung enthält
0,01 bis 10,0 Gew.-% eines Schneealgenextraktes aus *Chlamydocapsa* sp. (CCCryo 101-99, Stammsammlung IBMT), wobei die kultivierten Zellen mittels Hochdruckhomogenisierung aufgeschlossen und anschließend die Inhaltstoffe extrahiert werden und mit leeren Liposomen stabilisiert werden, um fein dispergierte Liposomen zu erhalten, die die fettlöslichen und wasserlöslichen Fraktionen der Zellen enthalten.
Neben diesen Bestandteilen sind pharmazeutisch und kosmetisch verträgliche Zusatzstoffe, wie Verdickungsmittel, Antioxidantien, pH-Stabilisatoren und dergleichen, vorhanden. Bevorzugt werden
(b) 0,01 bis 5 Gew.-% Verdickungsmittel
(c) 0,001 bis 1 Gew.-% Antioxidantien
(d) 0,001 bis 2 Gew.-% pH-Stabilisatoren
(e) 0,001 bis 2 Gew.-% Zusatzstoffe
verwendet.

### Beispiele

### Beispiel 1

### Produktion der Biomasse

Die Herstellung der Biomasse erfolgt in enger Zusammenarbeit mit dem IBMT Fraunhofer Institut Berlin in einem 2-Stufen Produktionsprozeß. Während der ersten Produktionsphase werden die Schneealgen in entsprechendem Kulturmedium (3N-BBM) bei einem pH-Wert von 5,5 angezogen und bei 4°C bis 15°C unter CO₂-Zufuhr kultiviert. Die Verdoppelungszeit der Algen beträgt ca. 1,3 Tage bei einer Temperatur von 14,5°C. Nach Abschluß der ersten Produktionsphase werden die Schneealgen in ein nährstofflimitiertes Kulturmedium übertragen um den Übergang in die Rotphase/stationäre Phase zu induzieren. Die Kultivierung in der 2. Produktionsphase wird mit denselben Parametern wie in der ersten Phase durchgeführt. Nach Beenden der 2. Produktionsphase wird die Zellbiomasse vom Kulturmedium abgetrennt und bei -20°C eingefroren.

### Beispiel 2

### Herstellung eines liposomalen Extraktes

Die gefrorene Biomasse wird aufgetaut, mit Konservierungsmittel und mit einer Dispersion, welche leere Liposomen einer Größe von rund 50 nm enthält, gemischt. Die Konzentration der Schneealgenbiomasse in der Mischung kann 0,01 g/l bis zu 10g/l betragen. Die Mischung wird dann viermal hochdruckhomogenisiert bei einem Druck von 1200 bar (1,2·10⁸ N m⁻²). Dabei werden die Zellwände der Schneealgen aufgebrochen, die fett- und wasserlöslichen Bestandteile des Cytosols freigesetzt und gleichzeitig in Form einer flüssigen Nanoemulsion eingekapselt. Der so hergestellte Extrakt enthält dispergierte und emulgierte Zellbestandteile. Mit dieser Technologie können unlösliche Lipidpartikel aus Zellbestandteilen und dem Cytosol bioverfügbar gemacht werden.

### Beispiel 3

### Trocknung des Extraktes mittels Wirbelschichttrocknung

Der so hergestellte und aufbereitete Schneealgenextrakt kann in verschiedenen Verhältnissen mit Trägermaterial gemischt werden. Idealerweise wird der Extrakt im Verhältnis 1/1 mit Isomalt/PVP gemischt. Andere Verhältnisse können zum Beispiel 1/2, 1/5 oder 1/10 sein. Als Trägermaterialien kommen verschiedene Substanzen wie zum Beispiel Maltose, Maltodextin, mikrokristalline Cellulose, silifizierte mikrokristalline Cellulose, Povidon oder andere Zuckerderivate auch in Kombination mit PVP in Frage. Der so vorbereitete Extrakt wird mittels Wirbelschichttrocknung mit einer Sprührate von 50 bis 60g/min und einer durchschnittlichen Temperatur von max. 50°C getrocknet bis auf einen Restfeuchte-Anteil von ca. 5% Wassergehalt. Die Temperaturführung und der Trocknungsgrad kann variabel eingestellt werden je nach gewünschter Granulatgröße und Schüttdichte des Endproduktes.

Die Prozentangaben, die in den Beispielen 4 bis 7 für die verwendeten Komponenten angegeben sind, beziehen sich auf die Gesamtmenge (w/w), es sei denn, es werden andere Angaben gemacht.

### Beispiel 4

### Antiphotoaging-Creme mit Schneealgenextrakt

**Tabelle 1**

| **Phase** | **Komponenten** | **% w/w** |
|---|---|---|
| **A** | Wasser | ad 100 |
| | Di-Natrium-EDTA | 0,1 |
| | Methylpropandiol | 2,0 |
| **B** | Cetylalcohol und Glycerolstearat und PEG-75 Stearat und Ceteth-20 und Steareth-20 | 4,0 |
| | Natrium-Acrylat-Copolymer and hydrogeniertes Polyisobuten und Phospholipid und Polyglycerol-10-Stearat and Helianthus Annuus (Sonnenblumen) Samenöl | 1,0 |
| | Stearylalcohol | 1,5 |
| | C₁₂₋₁₅ Alkylbenzoat | 4,0 |
| | 4-Hydroxybenzoesäureester in Phenoxyethanol | 0,7 |
| **C** | Cyclopentasiloxan und Dimethicon Kreuzpolymer | 3,0 |
| | Cyclotetrasiloxan und Cyclopentasiloxan | 4,0 |
| **D** | Schneealgenextrakt | 2,0 |
| **E** | Parfum | 0,1 |

Die in Tabelle 1 angegebenen Bestandteile werden, wie folgt beschrieben, zu einer Anti-Photoaging-Creme verarbeitet.

Die Komponenten der Phase A werden gemischt und auf 75°C erwärmt. Die Komponenten der Phase B werden ebenfalls gemischt und auf 75°C erwärmt.
Dann wird die Phase B unter Rühren zu Phase A gegeben und gemischt, bis die Masse homogen ist. Anschließend wird für 5 Minuten homogenisiert. Die erhaltende Masse wird unter Rühren auf 40°C abgekühlt. Die Komponenten der Phase C werden vorgemischt und zu der obigen Massen gegeben. Anschließend werden die Bestandteile der Phasen D und E zugegeben und ebenfalls gemischt. Die erhaltene Masse wird homogenisiert und anschließend unter Rühren auf Raumtemperatur abgekühlt.

Der pH-Wert sollte zwischen 5,5 bis 6,0 liegen. Andernfalls muss er mit Natronlauge oder Zitronensäure eingestellt werden.

### Beispiel 5

### Cell-Protecting-Serum mit Schneealgenextrakt

**Tabelle 2**

| **Phase** | **Komponenten** | **% w**/**w** |
|---|---|---|
| **A** | Octansäure/Decansäure-Triglyceride | 10,0 |
| | Glycerin- (und) Saccharaose-Laurat (und) Saccharose-Dilaurate (und) Saccharose-Trilaurate (und) Sorbitol | 2,0 |
| | Parfum | 0,12 |
| **(A/C)** | Dehydroacetsäure (und) Benzylalcohol (und) | 0,7 |
| | Wasser | |
| **B** | Glycerin | 15,0 |
| | Natriumpolyacrylat | 0,70 |
| **C** | Wasser | ad 100 |
| | Pentylenglycol | 5,0 |
| | Milchsäure 80% | 0,145 |
| **D** | Schneealgenextrakt | 5,0 |
| **E** | Siliciumdioxid-Dimethyl-Silylat | 0,1 |

Die in Tabelle 2 angegebenen Bestandteile werden, wie folgt beschrieben, zu einem Cell-Protecting-Serum verarbeitet.

Die Komponenten der Phase C werden mit der Hälfte des Konservierungsmittels (Dehydroacetsäure und Benzylalcohol) gemischt. Die Komponenten der Phase B werden gemischt und zu der Phase C zugegeben. Dann werden die Komponenten der Phase A gemischt und anschließend das restliche Konservierungsmittel zugegeben. Die Phase A wird unter Rühren zugegeben, und dann wird der Schneealgenextrakt ebenfalls unter Rühren zugegeben. Zum Schluß wird das Verdickungsmittel E unter Rühren eingestreut und die erhaltene Masse wird solange gemischt, bis alle Bestanteile homogen verteilt sind.

Der pH-Wert muss, falls er nicht zwischen 5,5 bis 6,0, liegt mit Natronlauge oder Zitronensäure eingestellt werden.

### Beispiel 6

### Anti-Aging-Hydrogel mit Schneealgenextrakt

**Tabelle 3**

| **Phase** | **Komponenten** | % w/w |
|---|---|---|
| **W1** | Wasser | ad 100 |
| | Glycerin | 2,5 |
| | Pentylenglycol | 5,0 |
| **W2** | Phenoxyethanol | 0,80 |
| **W3** | Hydroxyethylcellulose | 0,90 |
| **A** | Schneealgenextrakt | 2,0 |

Die in Tabelle 3 angegebenen Bestandteile werden, wie folgt beschrieben, zu einem Anti-Aging-Hydrogel verarbeitet.

Die Komponenten der Wasserphase W1 werden gemischt.
Das Phenoxyethanol der Phase W2 wird in der Wasserphase W1 gelöst. Anschließend wird das Verdickungsmittel W3 unter starkem Rühren in die erhaltene Wasserphase eingestreut und die erhaltene Lösung wird unter Rühren für 2 Stunden hydratisiert. Anschließend wird der Schneealgenextrakt zugegeben und die Mischung solange gemischt, bis sie homogen ist.

Der pH-Wert muss, falls er nicht zwischen 5,5 bis 6,0 liegt, mit Natronlauge oder Zitronensäure eingestellt werden.

### Beispiel 7

### Intensiv-Haarmaske mit Schneealgenextrakt

**Tabelle 4**

| **Phase** | **Komponenten** | **% w/w** |
|---|---|---|
| **W1** | Wasser | ad 100 |
| | Cetrimoniumchlorid | 0,70 |
| | Zitronensäure | 0,35 |
| | Hydroxyethylcellulose | 0,20 |
| **O1** | Stearamidopropyldimethylamin | 1,00 |
| | Cetearylalcohol | 5,00 |
| | Behentrimoniumchlorid | 2,00 |
| | Glycerolstearat | 1,50 |
| | Dicaprylyl Ether | 0,60 |
| **O2** | Bis(C₁₃₋₁₅Alkoxy) PG Amodimethicon | 0,30 |
| **P** | Parfum | q.s. |
| **A** | Schneealqenextrakt | 3,0 |

Die in Tabelle 4 angegebenen Bestandteile werden, wie folgt beschrieben, zu einer Intensiv-Haarmaske verarbeitet.

Die Komponenten der Phase O1 werden gemischt und auf 80°C erwärmt.
Die Komponenten der Phase W1 werden ebenfalls gemischt und auf 80°C erwärmt. Dann wird die Phase W1 zu der Phase O1 gegeben und für 2 Minuten homogenisiert. Anschließend wird die erhaltenen homogene Masse auf 30 bis 40°C abgekühlt und dann der Schneealgenextrakt zugegeben. Dann wird das Parfum und die Phase 02 zugefügt und die Masse für 2 Minuten homogenisiert. Zum Schluß läßt man die homogenisierte Masse auf Raumtemperatur abkühlen.

Der pH-Wert muss, falls er nicht zwischen 3,5 bis 4,5 liegt, mit Natronlauge oder Zitronensäure eingestellt werden.

Für die Beispiele 8 und 9 wurden NHDF (normal human dermal fibroblasts), die direkt aus der Haut isoliert wurden verwendet.

### Beispiel 8

### Modell mit natürlich gealterten humanen Fibroblasten Zellen

Fibroblasten der Passage 17 wurden bis zur Konfluenz in Medium inkubiert. Danach wurden die Zellen während 24 Stunden in Medium inkubiert, das 1 Gew.-% Schneealgen-Biomasse der vorliegenden Erfindung enthielt. Kontrollansätze waren Zellen der Passagen 17 und 8, die nur in Medium inkubiert wurden. Von allen Ansätzen wurde mittels quantitativer PCR (real time PCR) die Expression von 64 Alterungs-Markergenen untersucht.

**Tabelle 5**

| Gen | Rel. Expression P17 / P8 | Rel. Expression |
|---|---|---|
| | | P17 behandelt / |
| | | P17 unbehandelt |
| Kollagen 1 alpha 1 subunit | 0,46 | 1,63 |
| Kollagen 3 alpha 1 subunit | 0,58 | 3,4 |
| Interleukin-6 | 0,53 | 4,89 |
| Klotho | 0,43 | 3,41 |

### Beispiel 9

### Modell mit frühzeitig gealterten Fibroblasten Zellen

Fibroblasten der Passage 8 wurden in Medium inkubiert, das 1 Gew.% Schneealgen-Biomasse der vorliegenden Erfindung enthielt. Danach wurden die Zellen mit H₂O₂ behandelt (600 µM während 2 Stunden). Kontrollansätze waren Zellen der Passage 8 ohne H₂O₂-Behandlung und Zellen der Passage 8 mit H₂O₂-Behandlung in Medium allein. Von allen Ansätzen wurde mittels quantitativer PCR (real time PCR) die Expression von 64 Alterungs-Markergenen untersucht.

**Tabelle 6**

| Gen | Rel. Expression Kontrolle H₂O₂ zu Kontrolle ohne H₂O₂ | Rel. Expression H₂O₂ behandelt zu H₂O₂ unbehandelt |
|---|---|---|
| E2F-1 Transkriptionsfaktor | 1,56 | 0,52 |
| Fatty acid binding protein 3 | 2,52 | 0,48 |
| Insulin-like growth factor binding protein 7 | 1,42 | 0,45 |
| Leptin-Rezeptor | 4,47 | 0,64 |
| Matrix metalloproteinase 1 | 5,30 | 0,53 |
| Matrix metalloproteinase 3 | 10,24 | 0,60 |
| Tissue factor pathway inhibitor 2 | 17,92 | 0,44 |
| Thioredoxin | 1,72 | 0,53 |

### Beispiel 10

### Dermatologische Tests:

Eine Creme mit 3 Gew.-% Schneealgen-Wirkstoff wurde im Gesicht in einem Halbseitentest geprüft. Das Testprodukt wurde von 18 Frauen und 2 Männern während 3 Wochen zweimal täglich aufgetragen. Das Plazeboprodukt wurde jeweils auf der anderen Gesichtshälfte aufgetragen. Alle Probanden verbrachten die mittlere Woche in den Skiferien. Vor und nach der Behandlung wurde der transepidermalen Wasserverlust (TEWL) bestimmt und mit der VisioFace® (Courage + Khazaka) Apparatur die Hautfalten im Gesicht untersucht. In der mit dem Schneealgenwirkstoff behandelte Gesichtshälfte war der TEWL am Ende der Behandlung signifikant um 12% niedriger. Auf der Placeboseite war keine signifikante Änderung des TEWL meßbar. Die Auswertung der VisioFace® Resultate zeigten eine signifikante Auffüllung der Falten nach der Behandlung mit der Schneealgencreme. Auf der Placeboseite konnte keine signifikante Veränderung festgestellt werden.

## Patentansprüche

1. Kosmetische Zusammensetzung zur Verhinderung und Verzögerung der Hautalterung durch Stimulierung der Expression des Klotho-Gens, **dadurch gekennzeichnet, dass**
die Zusammensetzung 0,01 bis 10 Gew.-% eines Schneealgen-Extraktes enthält, wobei die Schneealgen vor der Verwendung von der vegetativen, grünen Phase in die stationäre, rot Phase überführt werden.

2. Kosmetische Zusammensetzung nach Anspruch 1, bei der der Schneealgen-Extrakt aus Schneealgen der Gattung Chlamydocapsa gewonnen wird,

3. Kosmetischen Zusammensetzung nach Anspruch 2, für die der Stamm Chlamydocapsa sp. 101-99 verwendet wird.

4. Kosmetischen Zusammensetzung nach einem der Ansprüche 1 bis 3, **dadurch gekennzeichnet, dass**
der Schneealgen-Extrakt liposomal verkapselt wird.

## Claims

1. Cosmetic composition for preventing and delaying skin aging by stimulating the expression of the Klotho gene, **characterized in that**
the composition contains 0.01 to 10 wt% of an extract of snow algae, wherein the snow algae are transferred before use from the vegetative green phase to the stationary red phase.

2. Cosmetic composition according to claim 1, wherein the snow algae extract is an extract of the snow algae genus Chlamydocapsa.

3. Cosmetic composition according to claim 2, for which the strain Chlamydocapsa sp. 101-99 is used.

4. Cosmetic composition according to claims 1 to 3, **characterized in that** the snow algae extract is encapsulated in liposomes.

## Revendications

1. Formulation cosmétique servant à empêcher et à retarder le vieillissement de la peau en stimulant l'expression du gène klotho,
**caractérisée en ce**
**que** la formulation contient 0,01 à 10 % en poids d'un extrait d'algues des neiges, sachant que les algues des neiges sont amenées, avant utilisation, de la phase verte végétative à la phase rouge stationnaire.

2. Formulation cosmétique selon la revendication 1, dans le cadre de laquelle l'extrait d'algues des neiges est obtenu à partir des algues des neiges de la famille des chlamydomonadaceae.

3. Formulation cosmétique selon la revendication 2, pour laquelle la souche chlamydomonadaceae sp. 101-99 est utilisée.

4. Formulation cosmétique selon l'une quelconque des revendications 1 à 3, **caractérisée en ce**
**que** l'extrait d'algues des neiges est encapsulé dans des liposomes.
